# EUROPEAN PATENT SPECIFICATION

(11) **EP 3 415 168 B1**
(45) Date of publication and mention of the grant of the patent: **19.08.2020**
(21) Application number: 18165288.4
(22) Date of filing: 30.03.2018
(51) Int. Cl.: A61L 2/26, A47L 15/50, B65G 65/00, B65G 9/00

(54) **TREATMENT MACHINE FOR OBJECTS, IN PARTICULAR FOR WASHING, THERMALLY DISINFECTING AND/OR STERILIZING OBJECTS**
BEHANDLUNGSMASCHINE FÜR OBJEKTE, INSBESONDERE ZUM WASCHEN, THERMISCHEN DESINFIZIEREN UND/ODER STERILISIEREN VON OBJEKTEN
MACHINE DE TRAITEMENT POUR OBJETS, EN PARTICULIER POUR LE LAVAGE, LA DÉSINFECTION THERMIQUE ET/OU LA STÉRILISATION D'OBJETS

(30) Priority: 31.03.2017 IT 201700035888
(43) Date of publication of application: 19.12.2018
(73) Proprietor: Steelco S.p.A., 31039 Riese Pio X (IT)
(72) Inventor: Capovilla, Ivone, 31037 Loria (TV) (IT)
(74) Representative: Petraz, Gilberto Luigi

(56) References cited:
- WO-A1-2015/145383
- GB-A- 904 657

## Description

### FIELD OF THE INVENTION

The present invention concerns a treatment machine for objects, for example a so-called "tunnel" treatment machine, in which successive steps of the treatment of objects can be carried out in one or more treatment chambers in which relative treatment zones are disposed one after the other.

By the term treatment it is possible to understand "in their entirety", both pretreatment operations of the objects, such as pre-washing, with hot or cold water and/or with detergents or other chemical products, and also washing operations proper, and also drying operations. Also included in the same term "treatment" are thermal disinfection, sterilization, generally in an autoclave, and decontamination by means of particularly aggressive and dangerous decontaminating substances, detergents and/or chemical agents, such as for example peracetic acid.

By way of example, the objects that can be treated in the treatment machine in question can be instruments used in the health sector, in the laboratory, for analysis or for research, instruments used in the pharmaceutical sector, or instruments in the medical sector, surgical instruments or comparable or similar instruments, without excluding, however, the application of the present invention to the treatment of objects in general.

### BACKGROUND OF THE INVENTION

It is known to make machines for treating objects, generally contained in one or more object-holder containers, such as racks or suchlike, which are fed inside at least one suitable treatment chamber, by feed means, for example a belt, where the desired treatment cycle is performed.

Normally, at least one door is provided, associated with an entrance aperture, which is mobile and driven by an aperture and closing mechanism to assume a high or open position in which it allows the entrance of the objects to be treated in the treatment chamber, and a low or closed position in which, in cooperation with fluidic sealing means, it seals the washing chamber from the outside.

In "single-door" treatment machines, the entrance and exit of the containers and of the treated objects contained therein take place through the same door. In "pass-through" treatment machines, two doors are provided, that is, one door associated with an entrance aperture and one door associated with an exit aperture, normally in a position opposite the entrance aperture, respectively for the entrance and exit of the containers and of the treated objects contained therein.

Inside the treatment chamber of the machine, one or more object-holder containers can be inserted. If several object-holder containers are inserted, they are positioned in sequence one after the other in the chamber.

In the case of a "single-door" treatment machine, a rollerway is provided on which the object-holder containers are rested and situated in correspondence with the door of the machine. From the door, therefore, the containers enter with the objects to be treated and the containers with the treated objects come out.

In the case of a "pass-through" treatment machine, generally two roller ways are provided, positioned on diametrically opposite sides of the machine, of which one side has an entrance door for the containers with objects to be treated and, the opposite side, has an exit door of the containers with the treated objects.

In particular, the treatment machines configured for sterilization are generally machines with a deep treatment chamber, for example even a few meters long, therefore, in such machines, multiple object-holder containers disposed in sequence are loaded. In the sterilization treatment relatively high temperatures are reached, for example up to about 140°C, therefore, it is good practice if the sterilization machine does not have, inside the treatment chamber, automatic movement means for the object-holder containers and/or sensors to detect their position.

A first disadvantage of known treatment machines, in particular sterilization machines, therefore, is the need to always provide for the manual movement of the object-holder containers, especially in the delicate step of their extraction from the treatment chamber. The movement step of the object-holder containers can be performed by gripping and sliding them, by operators responsible for this function.

The extraction of the object-holder container may also be necessary in the event of a premature interruption of a treatment cycle, for example due to a fault or alarm, both for "pass-through" machines and also for "single door" machines.

Document WO-A-2015/145383 describes a device to introduce and extract containers with respect to a sterilization machine in a direction of feed, comprising a container loading plane defining a movement path, and motorized linear guide members disposed along the container loading plane and configured to move the containers in a guided manner along the movement path in the direction of feed. The device also comprises an auxiliary guide extension connected to the container loading plane and mobile between a first retracted position, in which it is comprised in the bulk of the container loading plane, and a second operating position, in which it at least partly protrudes from the container loading plane to define an extension of the movement path.

Although it has undoubted advantages in moving the racks or containers in the sense that it prevents possible blockages thereof during movement, the device has only one attachment element, which can cause a certain instability during attachment and detachment maneuvers, especially for object-holder containers of a certain width, weight and/or, in general, bulk.

Moreover, the attachment device cannot be driven remotely and when desired or necessary, since the attachment element with which it is provided automatically interferes with the object-holder container, at least when it is extracted.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to provide a treatment machine for objects, in particular for washing, thermodisinfection and/or sterilization of objects, which can overcome at least one of the disadvantages of the state of the art.

One purpose of the present invention is therefore to provide a treatment machine for objects which allows at least to extract at least one object-holder container from a treatment chamber, preventing direct contact of the object-holder container with the operator or operators responsible for extraction.

Another purpose of the present invention is to provide a treatment machine for objects provided with at least one attachment device which can be engaged with any type of object-holder container, for example racks or suchlike.

Another purpose of the present invention is to provide a treatment machine for objects which has a device of a mainly mechanical type, able to allow the automatic attachment of at least one object-holder container at least when it is extracted.

Another purpose of the present invention is to provide a treatment machine for objects which is provided with an attachment device which can be commanded remotely and therefore conveniently and effectively by an operator who is not in the immediate vicinity of the treatment chamber, which can therefore be activated when desired and can engage in a desired section of the object-holder container.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with these purposes, a treatment machine, in particular for washing, thermally disinfecting and/or sterilizing objects according to the invention comprises at least a treatment chamber configured to house at least one object-holding container.

The machine comprises at least a mobile attachment device, positioned outside the treatment chamber and provided with one or more attachment elements able to be selectively activated to associate the attachment device in releasable mode with at least an element or part of the object-holding container, so as to move the object-holding container, by means of the attachment device, at least in an extraction step from the treatment chamber.

The object-holding container and the attachment device are housed on a slider; the attachment device comprises at least a sliding block provided with a frame rotatable around an axis of rotation and integrated with attachment elements; the attachment elements are able to be selectively activated or deactivated by means of at least an actuator element, configured to cooperate with the frame so as to allow its rotation, wherein the rotation alternately allows the activation or deactivation of the attachment elements; the actuator element is also associated with at least a shaft provided with suitable drive means positioned at the end of the slider opposite with respect to that which, during use, is situated in proximity to the treatment chamber.

Advantageously, therefore, in the present treatment machine, it is possible to perform efficient and stable attachment and detachment operations of the object-holding container, thanks to the use of a plurality of attachment elements and also the attachment device can be advantageously remotely driven by at least an operator, that is from one end of the slider, that, during use, is opposite the end of the slider positioned in proximity to the treatment chamber.

In the machine, the attachment device can be configured to be positioned with at least the attachment elements inserted in a compartment below the object-holding container.

The slider that houses the object-holding container and the attachment device, allows, for example, to be able to distance the object-holding container from the treatment zone, once the container has been extracted from the treatment chamber.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the specification or in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a three-dimensional view of a treatment machine, in particular for washing, thermally disinfecting and/or sterilizing objects according to the present invention;
- fig. 2 is another three-dimensional view of an attachment device of the present treatment machine in a step where it approaches an object-holding container to be extracted from a treatment chamber of the treatment machine;
- fig. 3 is a three-dimensional view of the attachment device in proximity to the object-holding container;
- fig. 4 is a three-dimensional view of the attachment device in a position able to allow the attachment of the object-holding container;
- fig. 5 is a three-dimensional view of the attachment device in an attachment position of the object-holding device;
- fig. 6 is a three-dimensional view of the attachment device in an extraction step of the object-holding container from the treatment chamber;
- figs. 7a and 7b are schematic lateral views in section that show respectively the detachment step and the attachment step of the object-holding container by the attachment device.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

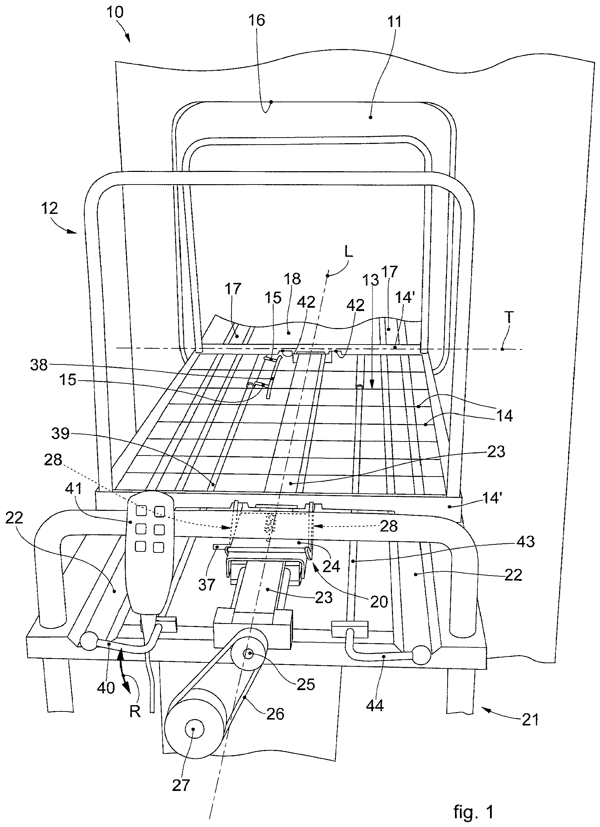

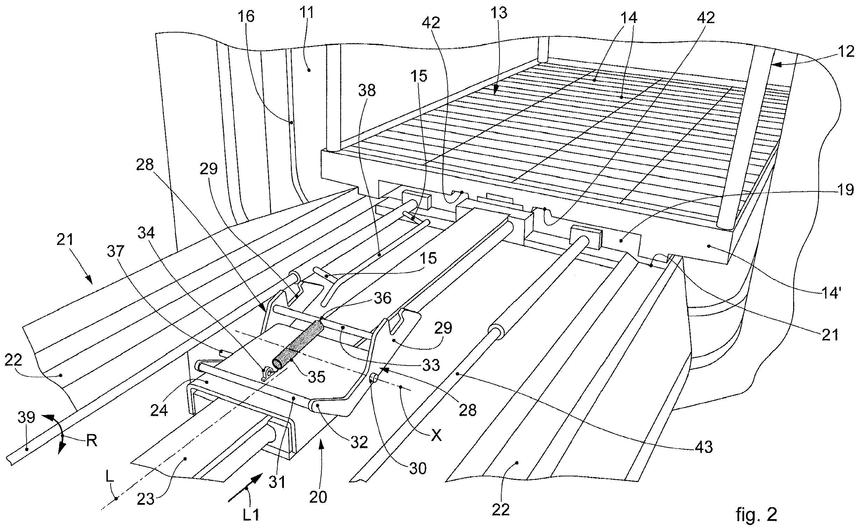

With reference to the attached drawings, in particular figs. 1 and 2, a treatment machine 10 comprises at least one treatment chamber 11 in which at least one object-holder container 12 can be positioned.

The object-holder container 12 could be in the form of a rack or suchlike and the objects to be treated in the chamber 11 are positioned therein.

Furthermore, more than one object-holder container 12 could be positioned in sequence in the treatment chamber 11.

Typically, the object-holder container 12 comprises a base 13 provided with a series of bars 14, disposed in a direction T substantially transverse with respect to a direction L for inserting and extracting the object-holder container 12 into and from the chamber 11.

Between the bars 14 at least two end bars 14' can be distinguished, which represent the head and the tail of the base 13 of the object-holder container 12.

The treatment chamber 11 will be provided inside it with means, not shown in the drawings, useful to carry out a specific treatment of the objects located in the object-holder container 12, for example washing, thermodisinfection, sterilization means or other.

The chamber 11 of the treatment machine 10 also comprises an aperture 16 to extract the object-holder container 12 with the treated objects.

The extraction aperture 16, in the case of a "single door" machine, will also be the aperture through which the object-holder container 12 is inserted with the objects to be treated.

In the case of a "pass-through" machine, an entrance aperture of the object-holder container 12 with the objects to be treated is provided, generally positioned on the side of the chamber 11 opposite the side where the extraction aperture 16 is positioned.

The aperture 16 will be opened or closed by means of a corresponding door, not shown in the drawings, which can be driven manually or automatically.

The opening and closing door of the aperture 16 may be a sliding door, for example a door with rolling shutter or suchlike, and is able to guarantee an adequate seal once the chamber 11 has been closed to carry out the treatment of the objects located in the object-holder container 12.

Inside the treatment chamber 11, the object-holder container 12 rests on a pair of supports 17, for example a pair of parallel tracks.

The supports 17 are made substantially in direction L on the base 18 of the treatment chamber 11.

The object-holder container 12 can also comprise wheels or suchlike to move them inside the chamber 11 in the direction L.

Between the base 18 of the treatment chamber 11 and the base 13 of the object-holder container 12 a compartment 19 is made, at least centrally, for the insertion of at least part of an attachment device 20 of the object-holder container 12.

The compartment 19 is therefore below the object-holder container 12 housed in the treatment chamber 11.

The attachment device 20 is preferably housed on a slider 21, mobile with respect to the treatment machine 10.

The slider 21 may be provided, for this purpose, with wheels or suchlike resting on the ground.

The slider 21 comprises at the sides at least a pair of supports 22, for example a pair of tracks, able to align, during use, with the supports 17 located on the base 18 of the treatment chamber 11, so as to be able to translate the object-holder container 12 from the treatment chamber 11 to the slider 21.

The slider 21 comprises, for example in a central zone, support and guide means for the attachment device 20.

The support means can be, for example, a guide 23, on which a block 24 of the attachment device 20 is slidably positioned.

The guide 23 can extend longitudinally over the entire length of the slider 21 and can carry, inside it, means to move the block 24 in the direction L.

For example, it is possible to provide, in a known manner, that the guide 23 is hollow inside and provided with a worm screw 25, directed longitudinally and able to engage with a female screw provided below the block 24.

The worm screw 25 can be connected, by means of suitable transmission elements 26, to drive means associated with a drive shaft 27, in short able to allow a bidirectional translation of the attachment device 20 along the guide 23.

At the sides of the block 24 a pair of arms 28 are provided, carrying attachment elements 29.

The pair of arms 28 provided with corresponding attachment elements 29, appropriately spaced, ensures greater stability and precision in the operation of attaching and detaching the object-holder container 12, compared with using a single attachment element.

The attachment elements 29 will be inserted into the compartment 19 below the object-holder container 12, so as to engage an element or part of the object-holder container 12.

The attachment elements 29 can be aligned in the same direction, for example the transverse direction T. In this case, the attachment elements 29 will be able to engage a single bar 14 or 14'.

The arms 28 are connected to the block 24 by means of a pin 30, which allows a certain oscillation thereof with respect to the block 24.

In the arms 28 it is possible to identify an attachment zone - carrying the attachment elements 29 - and a drive zone, configured to allow the arms 28 to move in the manner that will be explained in greater detail hereafter in the description.

The arms 28 are made integral with each other by means of a bar 31 which connects the ends 32 of the arms 28 opposite those of the attachment elements 29.

The arms 28 are also made integral with each other by means of an additional bar 33 which connects the arms 28 in a zone near to the attachment elements 29.

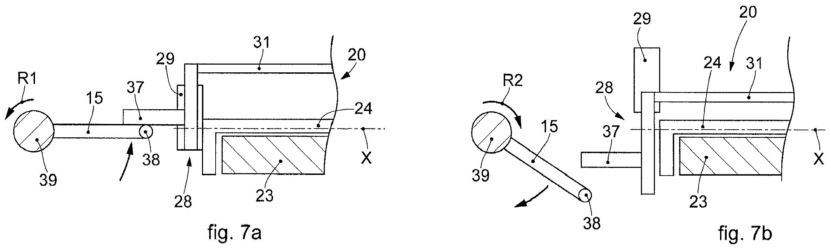

The pin 30 is substantially the fulcrum of the oscillation of the arms 28. For this reason, the pin 30 defines an axis of rotation X - indicated in figs. 2, 7a and 7b - around which the arms 28 can oscillate.

The bars 31 and 33 and the arms 28 thus form a frame, able to oscillate with respect to the pins 30 - and therefore with respect to the axis of rotation X - and able to allow, as we will see, the activation or deactivation of the attachment elements 29.

The arms 28 can be positioned substantially in direction L, while the bars 31 and 33 can be positioned substantially in direction T.

Moreover, the pins 30 could be replaced by a single pin 30 which passes through the block 24 transversely and engages both the arms 28, on one side and the other of the block 24.

A first end 34 of a spring 35, or other elastic return mean, is also attached on the surface of the block 24, the other end 36 of which is attached to the other bar 33.

The spring 35, or other elastic return means, keeps the attachment elements 29, at rest, in a raised position, as shown in fig. 2.

Naturally, it could be provided that the spring 35, or other elastic return means, keeps the attachment elements 29, at rest, in a lowered position, as an alternative to what has been shown.

At the side of at least one of the arms 28 a drive pin 37 is positioned, directed for example in a substantially transverse direction T.

The drive pin 37 is able to cooperate with an actuator element 38 connected to a shaft 39 by suitable connection elements 15, for example arms or suchlike, so that the actuator element 38 is in an offset position with respect to the shaft 39.

The actuator element 38 can be substantially parallel to the direction in which the shaft 39 is directed.

The direction in which the shaft 39 is directed can be the substantially longitudinal direction L.

The shaft 39, moreover, can extend substantially for the whole longitudinal extension of the slider 21.

The shaft 39 can be rotated in the direction R, see fig. 2, in one sense or the other, by means of suitable drive means 40.

The drive means 40 can be a manual lever, as shown in fig. 1.

Alternatively, the drive means 40 could be motorized means and connected to the shaft 39.

In fig. 1 a control panel 41 is also shown, which can be provided for example to manage and control the motion of the attachment device 20 and/or other elements of the treatment machine 10.

As can be seen, the drive means 40 of the shaft 39 are preferably located at the end of the slider 21 facing toward the operator, so as to be able to drive them conveniently and remotely.

In particular, the drive means 40 are therefore disposed at the end of the slider 21 opposite to that which, during use, is situated near the treatment chamber 11, see fig. 1 and fig. 2 for example.

The drive means 40 therefore allow to activate the attachment elements 29 in correspondence with any one of the bars 14 whatsoever of the object-holder container 12.

It is possible to provide that the shaft 39 is provided on one, the other or both sides of the attachment device 20, as schematically shown in fig. 1.

On the bar 14' of the object-holder container 12 seatings 42 can also be provided, able to facilitate the passage of the attachment elements 29 of the attachment device 20.

If we suppose that we have to extract the object-holder container 12 from the chamber 11, once the chamber 11 is opened, the situation could be like the one shown in fig. 2, that is to say with the bar 14' of the object-holder container 12 located in correspondence with the extraction aperture 16.

As we said, the extraction of the object-holder container 12, or of the object-holder containers 12, could be provided following the normal end of the treatment cycle, or it could be due to an interruption of the treatment cycle, to a malfunction, a breakdown, or other.

The slider 21, on which the attachment device 20 is positioned, is taken into proximity with the extraction aperture 16 as shown in fig. 1, then with the supports 22 in line with the supports 17 made on the base 18 of the treatment chamber 11.

As we said, the slider 21 can be provided with wheels or suchlike, for example rotating or tilting wheels, in order to be correctly positioned as described above.

It should be noted that, so that the slider 21 maintains the intended position with respect to the aperture 16, the slider 21 comprises an attachment device thanks to which, during use, the slider 21 remains constrained in position to the treatment machine 10.

The attachment device comprises a rod 43, which extends in the longitudinal direction L, substantially for the entire length of the slider 21.

The rod 43 is rotatable around its longitudinal extension axis. To this end, a drive lever 44 is provided which can be driven by the operator to constrain or release the slider 21 to/from the machine 10.

It should also be noted that a return mechanism, not shown, can be provided which connects the rod 43 to the shaft 39, making them integral with each other. At the end of the rod 43 and of the shaft 39 there are connection elements - not shown - which selectively allow to reciprocally constrain/release the slider 21 and the treatment machine 10.

The attachment device 20 is made to advance in direction LI, fig. 2, along the guide 23, until it moves into proximity with the object-holder container 12.

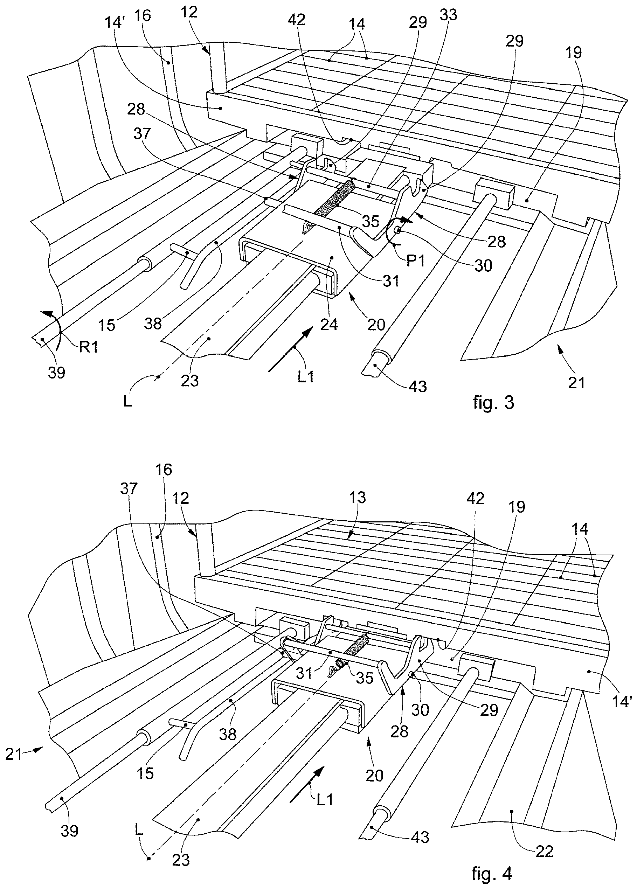

The shaft 39 is rotated in direction R1, see fig. 3 and fig. 7a, so that the actuator element 38 rises and lifts the drive pin 37 associated with the arm 28 which carries the drive pin 37.

The rotation R1 can be effected by acting on the drive means 40 of fig. 1 provided on the slider 21 and advantageously on the end of the slider 21 opposite the end nearest to the treatment chamber 11.

In fig. 3, in order to clearly show the drive pin 37 and its interaction with the actuator element 38, the arm 28 that carries the drive pin 37 and the bar 31 have been partly sectioned.

The lifting of the drive pin 37 causes the arms 28 to rotate around the axis of rotation X in a direction P1.

The rotation in direction P1 causes a lowering of the attachment elements 29, against the action of the spring 35, which tends to keep them in the raised position.

It should be noted that, so that the rotation in direction P1 determines the lowering of the attachment elements 29 as described, the pin 30 and the drive pin 37 must be offset with respect to each other in the longitudinal direction L, so that the pin 30 is nearer to the attachment elements 29 and that the drive pin 37 is nearer to the end 32 of the arms 28.

The spring 35, in fig. 3, is elongated with respect to the situation in fig. 2, in which it is at rest. This elongation, however, is not appreciable in fig. 3.

The attachment elements 29 are therefore in a lowered position and maintained in this position thanks to the actuator element 38 which keeps the drive pin 37 raised and the arm 28 appropriately rotated.

Continuing in the translation motion of the attachment device 20 in direction LI, see fig. 4, the attachment elements 29 are inserted into the compartment 19 made between the base 18 of the chamber 11 and the base 13 of the object-holder container 12.

The insertion is facilitated by the seatings 42 made in the bar 14' of the object-holder container 12.

The actuator element 38 must have an extension such as to keep the drive pin 37 engaged until the attachment elements 29 are correctly inserted under at least one element or part of the object-holder container 12, for example the head bar 14' of the object-holder container 12.

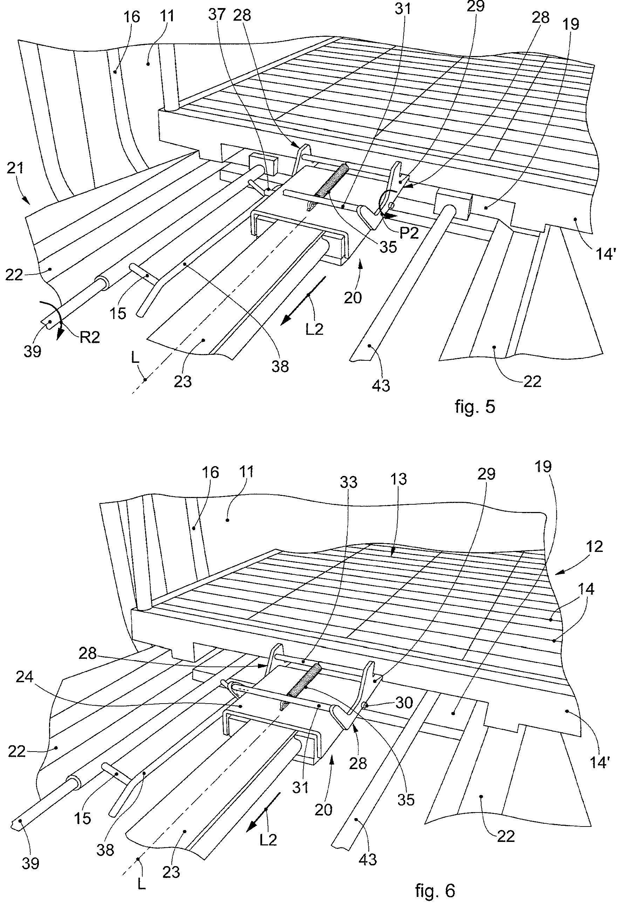

Once the attachment elements 29 are correctly positioned under an element or part of the object-holder container 12, for example the bar 14', see fig. 5, the shaft 39 is rotated in direction R2 by the drive means 40.

In fig. 5, in order to clearly show the drive pin 37 and the actuator element 38, the arm 28 that carries the drive pin 37 and the bar 31 have been partly sectioned.

In the schematic figs. 7a and 7b, moreover, the attachment element 29 has been shown with a greater width than the rest of the arm 28, in order to make it convenient and immediate to see.

The rotation of the rod in direction R2 causes the disengagement of the actuator element 38 from the drive pin 37, and therefore the automatic rotation of the arms 28 in direction P2, with consequent lifting of the attachment elements 29 into an active position.

The lifting of the attachment elements 29 takes place automatically thanks to the action of the spring 35, or other elastic return means, which returns again to the inactive position and tends, as we said, to keep the attachment elements 29 in a raised and active position.

The attachment device 20 will then return to the configuration shown for example also in fig. 2, this time however with the attachment elements 29 engaged with an element or part of the object-holder container 12, for example the bar 14'.

The attachment device 20 can then be translated in direction L2 along the guide 23, so as to proceed with the extraction of the object-holder container 12 from the treatment chamber 11, see also fig. 6.

The attachment device 20 can translate along the guide 23 so as to allow the complete extraction of the object-holder container 12 from the chamber 11 and hence its correct deposition on the slider 21.

The slider 21, with the object-holder container 12 above it, can be distanced from the zone in front of the chamber 11 of the treatment machine 10.

As an alternative to what has been shown, it would be possible to provide that the attachment device 20 can thrust itself farther inside the chamber 11, and/or be differently sized, so as to engage another bar 14 of the base 13 of the object-holder container 12, instead of the bar 14', or other element or part of the object-holder container 12.

As an alternative to what has been shown, more than two attachment elements 29 could be provided, for example in the case where the attachment device 20 were to have a width such as to advise the use of several attachment elements 29.

Moreover, the attachment elements 29 could also be disposed in positions not aligned with respect to the transverse direction T and, in this way, able to engage different elements or parts of the object-holder container 12, for example different bars 14 or 14' of the base 13 of the object-holder container 12.

As an alternative, moreover, the arms 28 and the bars 31 and 33, which substantially form a frame oscillating with respect to the block 24, could be replaced by another frame, structure or suchlike, able to allow the activation and deactivation of the attachment elements 29, so as to allow at least the extraction of the object-holder container 12 from the chamber 11 of the treatment machine 10.

It is also clear that, although the present invention has been described with reference to some specific examples, a person of skill in the art shall certainly be able to achieve many other equivalent forms of treatment machine for objects, in particular washing, thermal disinfection and/or sterilization of objects, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Treatment machine, for washing, thermally disinfecting and/or sterilizing objects, comprising at least a treatment chamber (11) configured to house at least a container (12), the treatment machine comprising at least a mobile attachment device (20), positioned outside said treatment chamber (11) and provided with a plurality of attachment elements (29) able to be selectively activated to associate said attachment device (20) in releasable mode with at least an element or part (14') of said container (12), so as to move said container (12), by means of said attachment device (20), at least in an extraction step from the treatment chamber (11), said object-holding container (12) and said attachment device (20) being housed on a slider (21), said attachment device (20) comprising at least a sliding block (24) provided with a frame (28, 31, 33) rotatable around an axis of rotation (X) and integrated with said attachment elements (29), said attachment elements (29) being able to be selectively activated or deactivated by means of at least an actuator element (38), configured to cooperate with said frame (28, 31, 33) so as to allow its rotation, said rotation alternately allowing the activation or deactivation of said attachment elements (29), the treatment machine being **characterized in that** said actuator element (38) is associated with at least a shaft (39) provided with suitable drive means (40) positioned at the end of said slider (21) opposite the end which, during use, is situated in proximity to the treatment chamber (11).

2. Machine as in claim 1, **characterized in that** said attachment device (20) is configured to be positioned with at least said attachment elements (29) inserted in a compartment (19) below said object-holding container (12).

3. Machine as in claim 1 or 2, **characterized in that** said attachment elements (29) engage on at least a bar (14') of said object-holding container (12) directed in a transverse direction (T) with respect to a longitudinal extraction direction (L) of the object-holding container (12).

4. Machine as in any claim hereinbefore, **characterized in that** said frame (28, 31, 33) is associated with elastic return means (35), configured to keep said attachment elements (29) automatically in at least an active or inactive position.

5. Machine as in any claim hereinbefore, **characterized in that** said frame (28, 31, 33) comprises at least a pair of arms (28) positioned at the sides of said sliding block (24) and provided with said attachment elements (29), at least one of said arms (28) being provided with at least a protruding drive pin (37) able to cooperate with said actuator element (38).

## Patentansprüche

1. Behandlungsvorrichtung zum Waschen, thermischen Desinfizieren und/oder Sterilisieren von Objekten mit wenigstens einer Behandlungskammer (11), die dazu eingerichtet ist, wenigstens einen Behälter (12) aufzunehmen, wobei die Behandlungsvorrichtung wenigstens eine bewegliche Befestigungsvorrichtung (20) aufweist, die außerhalb der Behandlungskammer (11) angeordnet und mit einer Anzahl von Befestigungselementen (29) ausgestattet ist, die dazu eingerichtet sind, wahlweise aktivierbar zu sein, um die Befestigungsvorrichtung (20) in einer lösbaren Art und Weise mit wenigstens einem Element oder einem Teil (14') des Behälters (12) zu verbinden, um den Behälter (12) mittels der Befestigungsvorrichtung (20) in wenigstens einem Entnahmeschritt von der Behandlungskammer (11) weg zu bewegen, wobei der objekthaltende Behälter (12) und die Befestigungsvorrichtung (20) von einem Schlitten (21) aufgenommen sind, wobei die Befestigungsvorrichtung (20) mit wenigstens einem Schiebeblock (24) ausgestattet ist, der über einen Rahmen (28, 31, 33) verfügt, der um eine Drehachse (X) drehbar und mit den Befestigungselementen (29) verbunden ist, wobei die Befestigungselemente (29) dazu eingerichtet sind, mittels wenigstens eines Betätigungselements (38), das dazu eingerichtet ist, mit dem Rahmen (28, 31, 33) zusammenzuwirken, um dessen Drehung zu gestatten, wobei die Drehung abwechselnd das Aktivieren oder Deaktivieren der Befestigungselemente (29) gestattet, aktivierbar oder deaktivierbar zu sein, wobei die Behandlungsvorrichtung **dadurch gekennzeichnet ist, dass** das Betätigungselement (38) mit wenigstens einer Welle (39) gekoppelt ist, die mit einem geeigneten Antriebsmittel (40) ausgestattet ist, das an dem Ende des Schlittens (21) angeordnet ist, das dem Ende gegenüberliegt, welches im Betrieb nahe der Behandlungskammer (11) angeordnet ist.

2. Vorrichtung nach Anspruch 1, **dadurch gekennzeichnet, dass** die Befestigungsvorrichtung (20) dazu eingerichtet ist, mit wenigstens den Befestigungselementen (29) positionierbar zu sein, die in einem Bereich (19) unterhalb des objekthaltenden Behälters (12) angeordnet sind.

3. Vorrichtung nach Anspruch 1 oder 2, **dadurch gekennzeichnet, dass** die Befestigungselemente (29) mit wenigstens einer Stange (14') des objektaufnehmenden Behälters (12) in Eingriff sind, die sich in einer Querrichtung (T) in Bezug auf eine Längsentnahmerichtung (L) des objektaufnehmenden Behälters (12) erstreckt.

4. Vorrichtung nach einem der vorangehenden Ansprüche, **dadurch gekennzeichnet, dass** der Rahmen (28, 31, 33) mit elastischen Rückzugmitteln (35) gekoppelt ist, die dazu eingerichtet sind, die Befestigungselemente (29) automatisch entweder in einer aktiven oder einer inaktiven Stellung zu halten.

5. Vorrichtung nach einem der vorangehenden Ansprüche, **dadurch gekennzeichnet, dass** der Rahmen (28, 31, 33) wenigstens ein Paar von Armen (28) aufweist, die an den Seiten des Schiebeblocks (24) angeordnet und mit den Befestigungselementen (29) ausgestattet sind, wobei wenigstens einer der Arme (28) mit wenigstens einem vorstehenden Antriebsstift (37) ausgestattet ist, der dazu eingerichtet ist, mit dem Betätigungselement (38) zusammenzuwirken.

## Revendications

1. Machine de traitement, pour laver, désinfecter thermiquement et / ou stériliser des objets, comprenant au moins une chambre de traitement (11) configurée pour loger au moins un contenant (12), la machine de traitement comprenant au moins un dispositif de fixation mobile (20), positionné à l'extérieur ladite chambre de traitement (11) et pourvu d'une pluralité d'éléments de fixation (29) pouvant être activés sélectivement pour associer ledit dispositif de fixation (20) en mode amovible à au moins un élément ou une partie (14') dudit contenant (12), de manière à déplacer ledit contenant (12), au moyen dudit dispositif de fixation (20), au moins dans une étape d'extraction à partir de la chambre de traitement (11), ledit contenant de confinement d'objet (12) et ledit dispositif de fixation (20) étant logés sur un coulisseau (21), ledit dispositif de fixation (20) comprenant au moins un bloc coulissant (24) muni d'un châssis (28, 31, 33) pouvant tourner autour d'un axe de rotation (X) et intégré avec lesdits éléments de fixation (29), lesdits éléments de fixation (29) pouvant être activés ou désactivés sélectivement au moyen d'au moins un élément d'actionnement (38), configuré pour coopérer avec ledit châssis (28, 31, 33) de manière à permettre sa rotation, ladite rotation permettant alternativement l'activation ou la désactivation desdits éléments de fixation (29), la machine de traitement étant **caractérisée en ce que** ledit élément d'actionnement (38) est associé à au moins un arbre (39) pourvu de moyens d'entraînement appropriés (40) positionnés à l'extrémité dudit coulisseau (21) opposé à l'extrémité qui, en utilisation, est située à proximité de la chambre de traitement (11).

2. Machine selon la revendication 1, **caractérisée en ce que** ledit dispositif de fixation (20) est configuré pour être positionné avec au moins lesdits éléments de fixation (29) insérés dans un compartiment (19) au-dessous ledit contenant de confinement d'objet (12).

3. Machine selon la revendication 1 ou 2, **caractérisée en ce que** lesdits éléments de fixation (29) viennent en prise sur au moins une barre (14') dudit contenant de confinement d'objet (12) dirigée dans une direction transversale (T) par rapport à une direction d'extraction longitudinale (L) du contenant de confinement d'objet (12).

4. Machine selon l'une quelconque des revendications précédentes, **caractérisée en ce que** ledit châssis (28, 31, 33) est associé à des moyens de rappel élastique (35), configurés pour maintenir automatiquement lesdits éléments de fixation (29) dans au moins une position active ou inactive.

5. Machine selon l'une quelconque des revendications précédentes, **caractérisée en ce que** ledit châssis (28, 31, 33) comprend au moins une paire de bras (28) positionnés sur les côtés dudit bloc coulissant (24) et pourvus desdits éléments de fixation (29), au moins l'un desdits bras (28) étant pourvu d'au moins une broche d'entraînement faisant saillie (37) apte à coopérer avec ledit élément d'actionnement (38).
